# EUROPEAN PATENT APPLICATION

(11) **EP 4 678 129 A1**
(43) Date of publication of application: **14.01.2026**
(21) Application number: 25188313.8
(22) Date of filing: 09.07.2025
(51) Int. Cl.: A61B 18/14, A61B 5/287, A61B 17/00, A61B 18/00

(54) **ELECTRODE HAVING RETENTION FEATURES FOR USE WITH A MEDICAL PROBE**

(30) Priority: 10.07.2024 US 202418768410
(71) Applicant: Biosense Webster (Israel) Ltd., 2066717 Yokneam (IL)
(72) Inventor: KEYES, Joseph Thomas, Irvine, 92618 (US); BEECKLER, Christopher Thomas, Irvine, 92618 (US); OKARSKI, Kevin Mark, Irvine, 92618 (US); HO, Thanh Tan, Irvine, 92618 (US)
(74) Representative: Carpmaels & Ransford LLP

(57) **Abstract**

The disclosed technology includes an electrode comprising a body extending along a longitudinal axis from a proximal end to a distal end. The body defines a tissue-facing surface, a lumen extending along the longitudinal axis through the body from the proximal end to the distal end, and one or more recesses extending through the body transverse to the longitudinal axis of the body. The one or more recesses are disposed between the tissue-facing surface and the lumen and define a first portion extending into the body a first depth and a second portion extending from the first portion a second depth into the body. The first portion comprises a first gap distance and the second portion comprises a second gap distance. The second gap distance can be greater than the first gap distance.

## Description

### Field

The present invention relates generally to medical devices, and in particular medical probes with electrodes, and further relates to, but not exclusively, medical probes suitable for mapping and ablation of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Regions of cardiac tissue can be mapped by a medical probe to identify the abnormal electrical signals. The same or different medical probe can be used to perform ablation. Some example probes include a number of spines with electrodes positioned thereon. The electrodes are generally attached to the spines and the spines are configured to bow radially outward when deployed from a sheath. The electrodes are then brought into contact with tissue for mapping or ablation of the tissue.

Some example catheters include a number of spines with electrodes positioned thereon. The electrodes are generally attached to the spines and secured in place by soldering, welding, or using an adhesive. It can be difficult to solder or weld electrodes to a spine due to their small size, however, and the adhesive generally does not adhere to the metal material as well as the solder or welding material. If the adhesive does not sufficiently adhere to the electrode or the adhesive delaminates from the electrode, the electrode can become loose on the spine and slide out of place along the spine. Attaching electrodes to spines using adhesive, however, is often an easier and more cost-effective process. What is needed, therefore, are systems and methods of attaching an electrode to a spine of a basket assembly using adhesive that ensures a sufficient bond to the electrode and the spine. These and other problems can be addressed by the technology disclosed herein.

### SUMMARY

There is provided, in accordance with the disclosed technology, an electrode for a medical probe. The electrode comprises a body extending along a longitudinal axis from a proximal end to a distal end. The body defines a tissue-facing surface, a lumen extending along the longitudinal axis through the body from the proximal end to the distal end, and one or more recesses extending through the body transverse to the longitudinal axis of the body. The one or more recesses are disposed between the tissue-facing surface and the lumen and define a first portion extending into the body a first depth and a second portion extending from the first portion a second depth into the body. The first portion comprises a first gap distance and the second portion comprises a second gap distance. The second gap distance can be greater than the first gap distance.

The disclosed technology further includes a medical probe comprising a tubular shaft including a proximal end and a distal end with the tubular shaft extending along a longitudinal axis of the tubular shaft. The medical probe further includes an expandable basket assembly coupled to the distal end of the tubular shaft. The expandable basket assembly can include a plurality of spines coupled to the tubular shaft and configured to bow radially outward from the longitudinal axis of the tubular shaft and a plurality of electrodes disposed on the plurality of spines. Each electrode of the plurality of electrodes can include a body extending along a longitudinal axis of the body from a proximal end to a distal end of the body. The body can define a tissue-facing surface, a lumen extending through the body from the proximal end to the distal end, and one or more recesses disposed between the tissue-facing surface and the lumen transverse to the longitudinal axis of the body. The one or more recesses can define a first portion extending into the body a first depth and a second portion extending from the first portion a second depth into the body. The first portion can comprise first gap distance and the second portion can comprise a second gap distance. The second gap distance can be greater than the first gap distance.

The disclosed technology further includes a method of manufacturing an electrode for a medical probe. The method can include forming a lumen through a body of an electrode with the lumen extending along a longitudinal axis from a proximal end to a distal end, and forming one or more recesses extending along the body transverse to the longitudinal axis. he one or more recesses can define a first portion extending a first depth into the body and a second portion extending from the first portion a second depth into the body. The first portion can include a first gap distance and the second portion can include a second gap distance. The second gap distance can be greater than the first gap distance.

### BRIEF DESCRIPTION OF THE DRAWINGS

The present disclosure will be more fully understood from the following detailed description of embodiments thereof, taken together with the drawings, in which:
FIG. 1 is a schematic pictorial illustration of a medical system including a medical probe with a distal end comprising electrodes, in accordance with the disclosed technology;
FIG. 2 is a schematic pictorial illustration of a basket catheter comprising electrodes, in accordance with the disclosed technology;
FIG. 3 is a perspective view of an electrode, in accordance with the disclosed technology;
FIG. 4A is a side view of the electrode, in accordance with the disclosed technology;
FIG. 4B is a detail view of the electrode of FIG. 4A, in accordance with the disclosed technology;
FIG. 5 is an end view of the electrode, in accordance with the disclosed technology;
FIG. 6 is a section view of the electrode of FIG. 5 taken along line A-A, in accordance with the disclosed technology; and
FIG. 7 is a flow chart illustrating a method of manufacturing a medical probe, in accordance with the disclosed technology.

### DETAILED DESCRIPTION

The electrodes described herein provide a solution to ensuring that electrodes are sufficiently secured in place on a spine when adhesive is used to secure the electrode in place. The electrodes include recesses in the body of the electrodes that are formed to receive adhesive but prevent the adhesive from being removed from the recess once the adhesive is hardened. The recesses, for example, include a first portion extending into the body of the electrode and a second portion extending from the first portion and having a gap distance that is larger than a gap distance of the first portion. In this way, the adhesive can enter the recesses when in a liquid form, but be prevented from being removed from the recesses when in a cured, solid form, thereby securing the electrode in place on the spine.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 70% to 110%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

The medical probe described herein can include electrodes that are configured to map electrophysiological signals propagating through tissue and/or deliver ablative energy to tissue. For example, the electrodes can be configured to deliver ablative energy to tissue using techniques such as bipolar or unipolar ablation using biphasic or monophasic signals as well as thermal ablative techniques. As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, methods or uses and devices that can be used for IRE ablation of cardiac tissue to treat cardiac arrhythmias. Ablative energies are typically provided to cardiac tissue by electrodes on an end effector of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

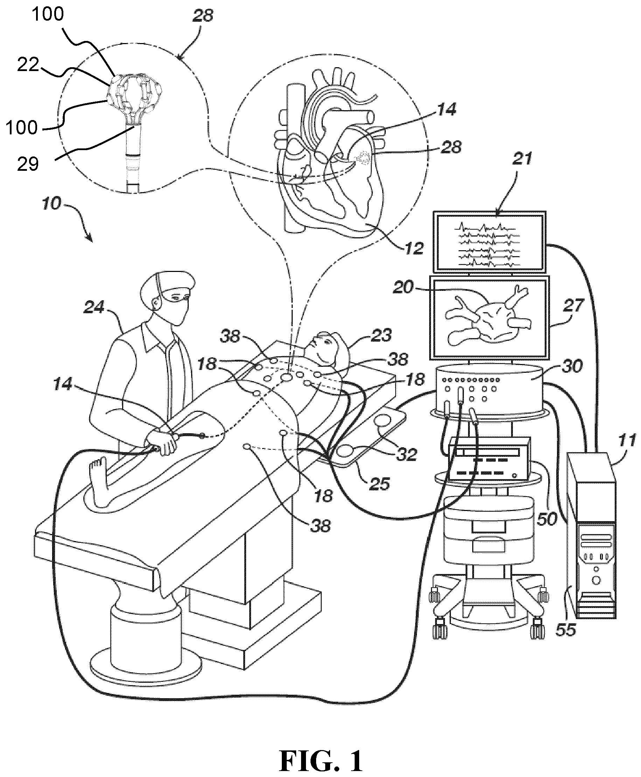

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing and ablating is illustrated herein. Physician 24 brings a distal tip 28 (sometimes referred to herein as basket catheter, basket assembly, expandable basket assembly, and/or end effector) of catheter 14 into contact with the heart wall for sensing a target site in heart 12.

Catheter 14 is an exemplary catheter that includes one and preferably multiple electrodes 100 optionally distributed over a plurality of spines 22 at distal tip 28 and configured to sense the IEGM signals and/or deliver ablative energy to tissue. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, the entireties of each of which are incorporated herein by reference.

System 10 includes one or more electrode patches 38 positioned for skin contact on patient 23 to establish location reference for location pad 25 as well as impedance-based tracking of electrodes 100. For impedance-based tracking, electrical current is directed toward electrodes 100 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, the entireties of each of which are incorporated herein by reference.

A recorder 11 displays electrograms 21 captured with body surface ECG electrodes 18 and intracardiac electrograms (IEGM) captured with electrodes 100 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

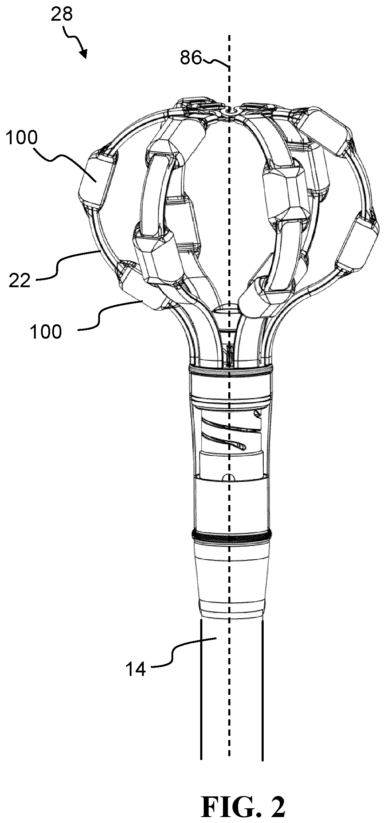

FIG. 2 is a schematic pictorial illustration showing a perspective view of a basket catheter 28 with spines 22 and electrodes 100 positioned in an expanded form, such as by being advanced out of a sheath (not shown). As shown in FIG. 2, the basket catheter 28 can include a plurality of spines 22 that can bow radially outward from a longitudinal axis 86 when in an expanded form. The spines 22 can be formed from a biocompatible resilient material such as nitinol such that the spines 22 are naturally biased to expand outwardly to the expanded form.

Each spine 22 can include one or more electrodes 100 attached thereto. The electrodes 100 can be configured such that the electrodes 100 can be disposed on the spines 22 in alignment on adjacent spines 22 or misaligned with electrodes 100 on adjacent spines 22. The electrodes 100 can be configured such that the electrodes can collapse together in a tight configuration when retracted into a sheath. The electrodes 100 described herein can be configured for mapping of electrophysiological signals through tissue and/or ablation of tissue.

Spines 22 may have elliptical (e.g., circular) or rectangular (that may appear to be flat) cross-sections, and include a flexible, resilient material (e.g., a shape-memory alloy such as nickel-titanium, also known as Nitinol or other materials such as cobalt chromium, stainless steel, titanium, or even a polymer material) forming the spine 22. The disclosed technology can be applicable to a basket catheter 28 formed from a single spine 22 or multiple spines 22 with each spine 22 being attached at both ends.

The spines 22 can be formed from a single sheet of planar material. In some examples, the spines 22 can be formed from the single sheet of planar material such that the spines 22 converge toward a central intersection. In other examples, the spines 22 can be formed individually and then attached at both ends as shown and just described.

As will be appreciated, the spine 22 can be electrically isolated from the electrode 100 to prevent arcing from the electrode 100 to the spine 22. For example, insulative jackets (not shown) can be positioned between the spine 22 and the electrode 100, but one of skill in the art will appreciate that other insulative coverings are contemplated. For example, an insulative coating can be applied to the spine 22, the electrodes 100, or both. The insulative jackets can be made from a biocompatible, electrically insulative material such as polyamidepolyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets may also include one or more additives or fillers, such as, for example, polytetrafluoroethylene (PTFE), boron nitride, silicon nitride, silicon carbide, aluminum oxide, aluminum nitride, zinc oxide, and the like.

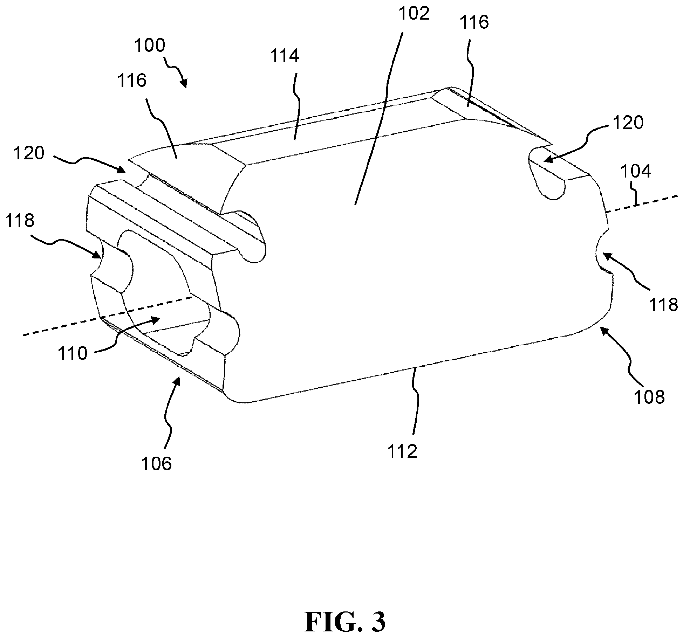

FIG. 3 is a perspective view of an electrode 100, in accordance with the disclosed technology. The electrode 100 can include a body 102 that extends along a longitudinal axis 104 from a proximal end 106 to a distal end 106. The electrode 100 can be formed in various shapes, sizes, and configurations depending on the particular application. As shown, the electrode 100 can define a lumen 110 extending through the body 102 along the longitudinal axis 104. The lumen 110 can be configured to receive a spine 22 of the basket catheter 28 such that the electrode 100 can be slid along the spine 22 and secured to the spine 22.

The electrode 100 can include an inwardly-facing surface 112 and a tissue-facing surface 114. For example, the electrode 100 can be configured such that, when the electrode 100 is attached to a spine 22, the inwardly-facing surface 112 can be a surface of the electrode 100 that faces inwardly into a central portion of the basket assembly 28 while the tissue-facing surface 114 can be a surface of the electrode 100 that faces outwardly from the basket assembly 28 such that the tissue-facing surface 114 is configured to contact tissue when deployed within the patient 23. To help ensure the basket assembly 28 is atraumatic, the electrode 100 can include rounded edges 116 (atraumatic edges) at least on surfaces that are likely to contact tissue. The rounded edges 116, as will be described in greater detail herein, can be formed, for example, by tumbling, sanding, filing, deburring, sand blasting, or other methods of rounding an edge of the electrode 100.

All or some of the electrode 100 can comprise a conductive material. For example, all of the electrode 100 can be made entirely from gold, silver, platinum, palladium, stainless steel (and their respective alloys). Alternatively, only a portion of the electrode 100 (e.g., the tissue-facing surface 114) can be made from the just-recited materials. These materials also have high thermal conductivity which allows the minimal heat generated on the tissue (i.e., by the ablation energy delivered to the tissue) to be conducted through the electrodes 100 to the back side of the electrodes 100 (i.e., the inwardly-facing surfaces 112), and then to the blood pool in heart 12. The electrodes can be configured to deliver electrical pulses having a peak voltage of at least 900 volts (V).

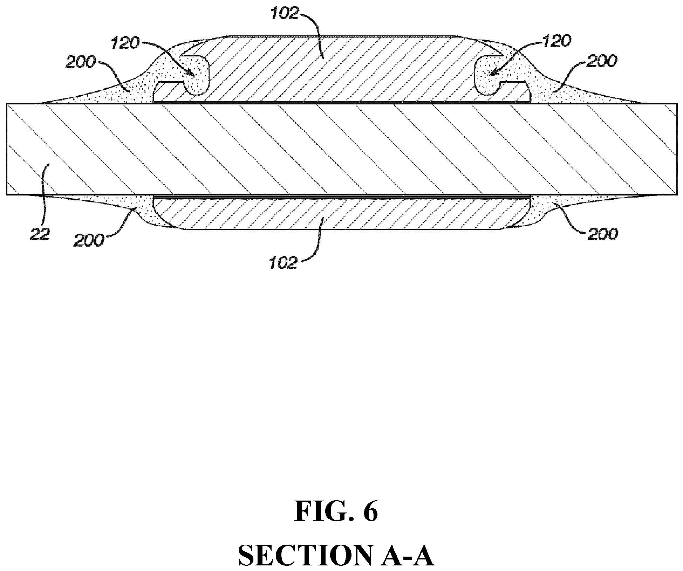

The electrode 100 can include one or more end recesses 118 with one positioned near the proximal end 106 and another positioned near the distal end 108. The end recesses 118 can be positioned along the longitudinal axis 104 and align with the lumen 110 that extends through the body 102. The end recesses 118 can be configured to receive at least a portion of an adhesive 200 (described in more detail in relation to FIG. 6) that is positioned over the electrode 100 and the spine 22. The end recesses 118 can help provide more surface area for the adhesive to adhere to the electrode 100. The end recesses 118 can help to prevent the electrode 100 from rotating about the longitudinal axis 104 to help ensure the tissue-facing surface 114 is correctly positioned to contact tissue. In other words, the end recesses 118 can be configured to receive at least a portion of the adhesive 200 and partially interlock with the adhesive 200 acting as a mechanical lock preventing the electrode 100 from rotating about the longitudinal axis 104. Additionally, or alternatively, the end recesses 118 can also be configured to receive one or more wires such that the wires can be adhered or welded in place.

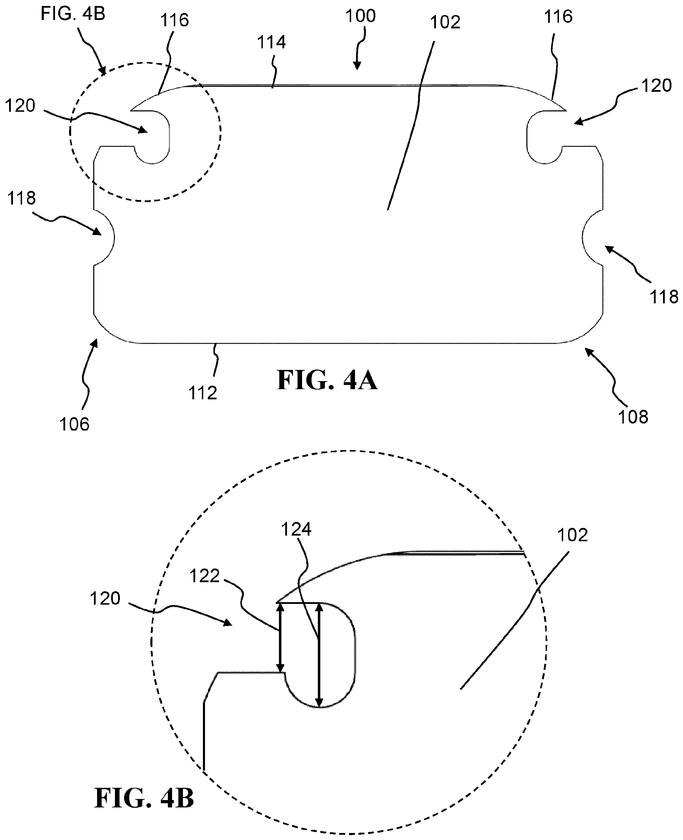

The electrode 100 can further include one or more recesses 120 positioned at the proximal end 106 and the distal end 108 at a position on the electrode 100 that is at least partially between the tissue-facing surface 114 and the lumen 110. FIG. 4A is a side view of the electrode 100 while FIG. 4B is a detail view of the electrode 100 of FIG. 4A, in accordance with the disclosed technology. As shown, the recesses 120 can include a first gap distance 122 nearest an outer portion of the recess 120 and a second gap distance 124 at an inner portion of the recess 120. The second gap distance 124 can be greater than the first gap distance 122. That is, a first portion of the recess 120 can extend into the body 102 of the electrode 100 a first depth having a first gap distance 122 and a second portion of the recess 120 can extend from an end of the first portion into the body 102 a second depth having a second gap distance 124 that is greater than the first gap distance 122. In this way, the recesses 120 can be configured to receive adhesive 200 while the adhesive 200 is in a fluid (or semi-fluid) state and then prevent the adhesive 200 from being removed from the recess 120 once cured. That is, once the adhesive 200 becomes a solid material, the adhesive 200 that is in the second portion having the second gap distance 124 will be unable to be pulled back through the first portion which has the smaller first gap distance 122. That is, the adhesive 200 can form a bayonet connection with the electrode 100 via the recesses 120 to help ensure the electrode 100 is secured to the spine 22.

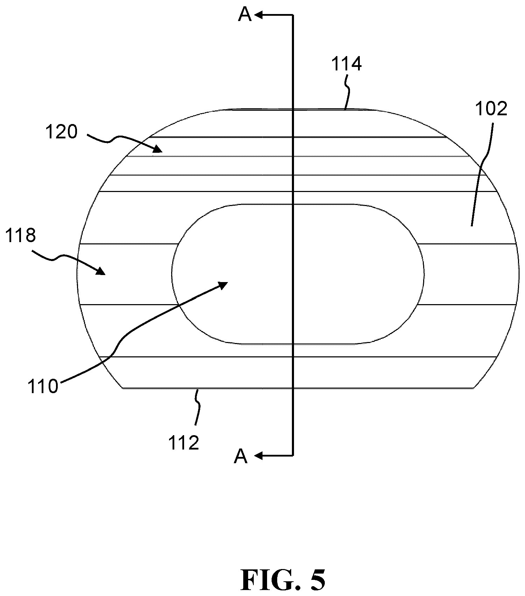

FIG. 5 is an end view of the electrode 100 while FIG. 6 is a section view of the electrode 100 of FIG. 5 taken along line A-A, in accordance with the disclosed technology. As shown in FIG. 5, the electrode 100 can include a rounded profile, further helping the electrode 100 to form an atraumatic shape. In some examples, the tissue-facing surface 114 can have a smaller surface area than the inwardly-facing surface 112 while in other examples, the tissue-facing surface 114 can have a great surface area or an equal surface area compared with the inwardly-facing surface 112.

As shown more clearly in FIG. 6, the adhesive 200 can be disposed at least partially over the electrode 100 and the spine 22 to secure the electrode 100 to the spine 22. The recesses 120 can be configured to receive at least a portion of the adhesive 200, thereby preventing the adhesive 200 from becoming delaminated from the electrode 100. As shown, the adhesive 200 can extend around the spine 22 and the electrode 100 on the top, bottom, and sides (not visible in FIG. 6) to help secure the electrode 100 to the spine 22. In this way, the adhesive 200 can be at least partially disposed in the recesses 120 and the end recesses 118

The adhesive 200 can be a non-conductive material that can be initially in a liquid or non-solid form and then harden into a solid or semi-solid form, thereby securing the electrode 100 to the spine 22. In this way, the adhesive 200 can be flowed into the recesses 120 when installed and then allowed to harden, thereby preventing the adhesive 200 from delaminating from the electrode 100 or otherwise becoming unattached to the electrode 100. The adhesive 200 can be a polymer material or other biocompatible adhesive and be a naturally-curing adhesive, a light-curing adhesive, a heat-curing adhesive (including a heat-curing silicone adhesive), an ultraviolet- (UV) light-curing adhesive, a cyanoacrylate adhesive, an acrylic, an epoxy, polymethyl methacrylate (PMMA), fibrin, a polyurethane adhesive, reflowed polymer (such as polyurethane, polyetheretetherketone (PEEK), or polyethelene terephthalate (PET)), or other suitable types of adhesives.

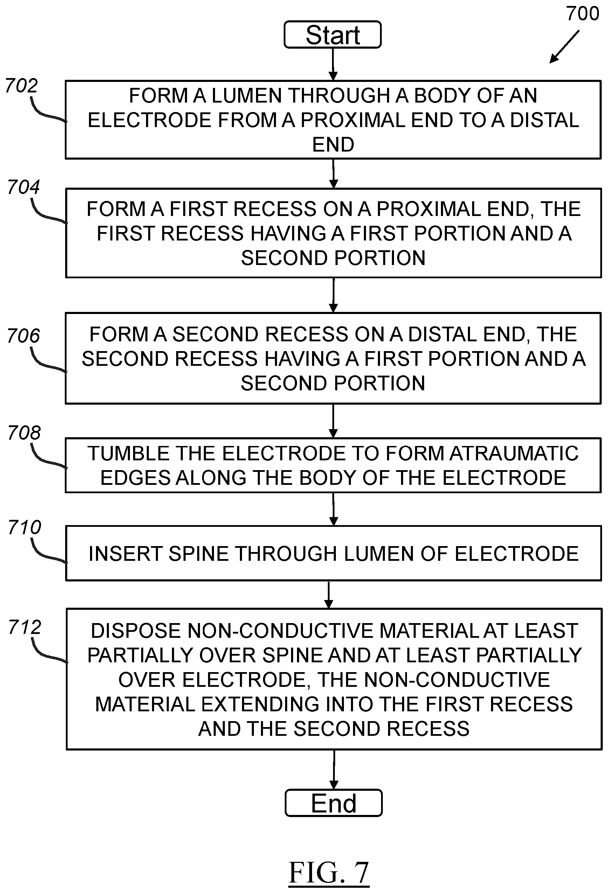

FIG. 7 is a flow chart illustrating a method 700 of manufacturing a medical probe, in accordance with the disclosed technology. The method 700 can include forming 702 a lumen through a body of an electrode from a proximal end to a distal end of the electrode. The method 700 can include forming 704 a first recess on the proximal end of the electrode and forming 706 a second recess on a distal end of the electrode. The first recess and the second recess can include a first portion and a second portion in accordance with the recesses 120 described in greater detail herein.

The method 700 can further include tumbling 708 the electrode to for atraumatic edges along the body of the electrode and then inserting 710 a spine through the lumen of the electrode. The method 700 can include disposing 712 non-conductive material (e.g., adhesive as described more fully herein) at least partially over the spines and at least partially over the electrode. The non-conductive material can extend into the first recess and the second recess and described more fully herein. In this way, the electrode 100 can be secured to the spine and prevented from sliding along the length of the spine.

The disclosed technology described herein can be further understood according to the following clauses:
Clause 1: An electrode for a medical probe, the electrode comprising: a body extending along a longitudinal axis from a proximal end to a distal end, the body defining: a tissue-facing surface; a lumen extending along the longitudinal axis through the body from the proximal end to the distal end; and one or more recesses extending through the body transverse to the longitudinal axis of the body, the one or more recesses disposed between the tissue-facing surface and the lumen and defining: a first portion extending into the body a first depth, the first portion comprising a first gap distance; and a second portion extending from the first portion a second depth into the body, the second portion comprising a second gap distance, the second gap distance being greater than the first gap distance.
Clause 2: The electrode of clause 1, the lumen configured to receive a spine of the medical probe.
Clause 3: The electrode of any one of the preceding clauses, wherein the one or more recesses are configured to receive a non-conductive material disposed at least partially over the spine and the electrode.
Clause 4: The electrode of any one of the preceding clauses, the body further defining an inwardly-facing surface, the one or more recesses being positioned nearer the tissue-facing surface than the inwardly-facing surface.
Clause 5: The electrode of clause 4, the one or more recesses comprising a first recess disposed nearest the distal end of the body and a second recess disposed nearest the proximal end of the body.
Clause 6: The electrode of any one of the preceding clauses, the body further defining one or more atraumatic edges.
Clause 7: The electrode of any one of the preceding clauses, wherein the tissue-facing surface is configured to deliver ablative energy to tissue.
Clause 8: The medical probe of clause 7, wherein the tissue-facing surface is configured to deliver electrical pulses for irreversible electroporation, the pulses having a peak voltage of at least 900 volts (V).
Clause 9: A medical probe, comprising: a tubular shaft including a proximal end and a distal end, the tubular shaft extending along a longitudinal axis of the tubular shaft; and an expandable basket assembly coupled to the distal end of the tubular shaft, the expandable basket assembly comprising: a plurality of spines coupled to the tubular shaft and configured to bow radially outward from the longitudinal axis of the tubular shaft; and a plurality of electrodes disposed on the plurality of spines, each electrode of the plurality of electrodes comprising: a body extending along a longitudinal axis of the body from a proximal end to a distal end of the body, the body defining a tissue-facing surface, a lumen extending through the body from the proximal end to the distal end, and one or more recesses disposed between the tissue-facing surface and the lumen transverse to the longitudinal axis of the body, the one or more recesses defining: a first portion extending into the body a first depth, the first portion comprising a first gap distance; and a second portion extending from the first portion a second depth into the body, the second portion comprising a second gap distance, the second gap distance being greater than the first gap distance.
Clause 10: The medical probe of clause 9, the lumen configured to receive a respective spine of the plurality of spines.
Clause 11: The medical probe of any one of clauses 9 or 10, wherein the one or more recesses are configured to receive a non-conductive material disposed at least partially over the electrode and a respective spine of the plurality of spines to secure the electrode to the respective spine.
Clause 12: The medical probe of any one of clauses 9 to 11, the body of each electrode of the plurality of electrodes further defining an inwardly-facing surface, the one or more recesses being positioned nearer the tissue-facing surface than the inwardly-facing surface.
Clause 13: The medical probe of clause 12, wherein the inwardly-facing surface is configured to face an inner portion of the expandable basket assembly when the plurality of electrodes are disposed on the plurality of spines.
Clause 14: The medical probe of clause 13, the tissue-facing surface configured to face outwardly from the inner portion of the expandable basket assembly to contact tissue.
Clause 15: The medical probe of any one of clauses 9 to 14, the one or more recesses comprising a first recess disposed nearest the distal end of the body and a second recess disposed nearest the proximal end of the body.
Clause 16: The medical probe of any one of clauses 9 to 15, the body of each electrode of the plurality of electrodes further defining one or more atraumatic edges.
Clause 17: The medical probe of any one of clauses 9 to 16, wherein each electrode of the plurality of electrodes is configured to deliver ablative energy to tissue.
Clause 18: The medical probe of any one of clauses 9 to 17, wherein the plurality of electrodes is configured to deliver electrical pulses for irreversible electroporation, the pulses having a peak voltage of at least 900 volts (V).
Clause 19: A method of manufacturing an electrode for a medical probe, the method comprising: forming a lumen through a body of an electrode, the lumen extending along a longitudinal axis from a proximal end to a distal end; and forming one or more recesses extending along the body transverse to the longitudinal axis, the one or more recesses defining: a first portion extending a first depth into the body, the first portion comprising a first gap distance; and a second portion extending from the first portion a second depth into the body, the second portion comprising a second gap distance, the second gap distance being greater than the first gap distance.
Clause 20: The method of clause 19 further comprising tumbling the electrode to form atraumatic edges.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An electrode for a medical probe, the electrode comprising:
a body extending along a longitudinal axis from a proximal end to a distal end, the body defining:
a tissue-facing surface;
a lumen extending along the longitudinal axis through the body from the proximal end to the distal end; and
one or more recesses extending through the body transverse to the longitudinal axis of the body, the one or more recesses disposed between the tissue-facing surface and the lumen and defining:
a first portion extending into the body a first depth, the first portion comprising a first gap distance; and
a second portion extending from the first portion a second depth into the body, the second portion comprising a second gap distance, the second gap distance being greater than the first gap distance.

2. The electrode of claim 1, the lumen configured to receive a spine of the medical probe.

3. The electrode of claim 2, wherein the one or more recesses are configured to receive a non-conductive material disposed at least partially over the spine and the electrode.

4. The electrode of claim 1, the body further defining an inwardly-facing surface, the one or more recesses being positioned nearer the tissue-facing surface than the inwardly-facing surface.

5. The electrode of claim 1, the body further defining one or more atraumatic edges.

6. The electrode of claim 1, wherein the tissue-facing surface is configured to deliver ablative energy to tissue, optionally wherein the tissue-facing surface is configured to deliver electrical pulses for irreversible electroporation, the pulses having a peak voltage of at least 900 volts (V).

7. A medical probe, comprising:
a tubular shaft including a proximal end and a distal end, the tubular shaft extending along a longitudinal axis of the tubular shaft; and
an expandable basket assembly coupled to the distal end of the tubular shaft, the expandable basket assembly comprising:
a plurality of spines coupled to the tubular shaft and configured to bow radially outward from the longitudinal axis of the tubular shaft; and
a plurality of electrodes disposed on the plurality of spines, each electrode of the plurality of electrodes comprising:
a body extending along a longitudinal axis of the body from a proximal end to a distal end of the body, the body defining a tissue-facing surface, a lumen extending through the body from the proximal end to the distal end, and one or more recesses disposed between the tissue-facing surface and the lumen transverse to the longitudinal axis of the body, the one or more recesses defining:
a first portion extending into the body a first depth, the first portion comprising a first gap distance; and
a second portion extending from the first portion a second depth into the body, the second portion comprising a second gap distance, the second gap distance being greater than the first gap distance.

8. The medical probe of claim 7, the lumen configured to receive a respective spine of the plurality of spines.

9. The medical probe of claim 7, wherein the one or more recesses are configured to receive a non-conductive material disposed at least partially over the electrode and a respective spine of the plurality of spines to secure the electrode to the respective spine.

10. The medical probe of claim 7, the body of each electrode of the plurality of electrodes further defining an inwardly-facing surface, the one or more recesses being positioned nearer the tissue-facing surface than the inwardly-facing surface, optionally wherein the inwardly-facing surface is configured to face an inner portion of the expandable basket assembly when the plurality of electrodes are disposed on the plurality of spines, further optionally the tissue-facing surface configured to face outwardly from the inner portion of the expandable basket assembly to contact tissue.

11. The electrode of claim 4 or the medical probe of claim 7, the one or more recesses comprising a first recess disposed nearest the distal end of the body and a second recess disposed nearest the proximal end of the body.

12. The medical probe of claim 7, the body of each electrode of the plurality of electrodes further defining one or more atraumatic edges.

13. The medical probe of claim 7, wherein each electrode of the plurality of electrodes is configured to deliver ablative energy to tissue, or the plurality of electrodes is configured to deliver electrical pulses for irreversible electroporation, the pulses having a peak voltage of at least 900 volts (V).

14. A method of manufacturing an electrode for a medical probe, the method comprising:
forming a lumen through a body of an electrode, the lumen extending along a longitudinal axis from a proximal end to a distal end; and
forming one or more recesses extending along the body transverse to the longitudinal axis, the one or more recesses defining:
a first portion extending a first depth into the body, the first portion comprising a first gap distance; and
a second portion extending from the first portion a second depth into the body, the second portion comprising a second gap distance, the second gap distance being greater than the first gap distance.

15. The method of claim 14 further comprising tumbling the electrode to form atraumatic edges.
